# EUROPÄISCHE PATENTSCHRIFT

(11) **EP 1 142 571 B1**
(45) Veröffentlichungstag und Bekanntmachung des Hinweises auf die Patenterteilung: **10.09.2008**
(21) Anmeldenummer: 01108608.9
(22) Anmeldetag: 05.04.2001
(51) Int. Cl.: A61K 31/195, A61K 9/20

(54) **Verfahren zur Herstellung von festen Kreatin-Dosierungsformen und dadurch erhältliche Dosierungsformen**
Method to produce solid creatine dosage forms and dosage forms thus obtained
Procédé pour la production de formes galéniques solides à base de créatine et formes galéniques ainsi obtenues

(30) Priorität: 06.04.2000 DE 10017102
(43) Veröffentlichungstag der Anmeldung: 10.10.2001
(73) Patentinhaber: Ocean Pharma GmbH, 21465 Reinbek (DE)
(72) Erfinder: Bogenstätter, Thomas, 67098 Bad Dürkheim (DE); Scherr, Günter, 67065 Ludwigshafen (DE); Kessel, Knut, 68161 Mannheim (DE); Breitenbach, Jörg, 68199 Mannheim (DE); Berndl, Gunther, 67273 Herxheim (DE)
(74) Vertreter: Kinzebach, Werner

(56) Entgegenhaltungen:
- WO-A-99/00122
- DE-A- 19 537 494
- DE-A- 19 729 305
- CHEMICAL ABSTRACTS, vol. 103, no. 14, 7. Oktober 1985 (1985-10-07) Columbus, Ohio, US; abstract no. 109936, MORISHITA PHARMACEUTICAL CO., LTD., JAPAN: "Pharmaceuticals containing creatine and its precursors for treatment of renal disorders" XP002171918 & JP 60 054320 A (MORISHITA PHARMACEUTICAL CO., LTD., JAPAN) 28. März 1985 (1985-03-28)

## Beschreibung

Die Erfindung betrifft ein Verfahren zur Herstellung von festen Kreatin enthaltenden Dosierungsformen und die dadurch erhältlichen Dosierungsformen.

Das Aminosäure-Derivat Kreatin kommt in der Natur insbesondere als Kreatinphosphat im Wirbeltiermuskel vor. Kreatinphosphat dient hier als Energieträger der Zelle für muskuläre Kontraktionsenergie. Kreatin wird aus der Nahrung aufgenommen oder endogen in Pankreas und Leber synthetisiert. Es kann aus natürlichen Quellen isoliert oder durch Guanylierung von Sarkosin synthetisiert werden. Kreatin findet als Nahrungsergänzung Einsatz bei der Therapie von neuromuskulären Erkrankungen (z.B. Muskeldystrophie) und Endokrinopathien, bei denen es zu einer mangelhaften Kreatinspeicherung und vermehrter -Ausscheidung über den Urin kommt. Neben der Verwendung in Kulturmedien und als Geschmacksverstärker in Gewürzen wird Kreatin in zunehmendem Maße als Nahrungsergänzungsstoff im Sport zur Steigerung der körperlichen Leistungsfähigkeit und insbesondere beim Bodybuilding eingesetzt.

Kreatin kommt in Form eines unter Umgebungsbedingungen stabilen Monohydrats vor. In den bekannten pharmazeutischen Zubereitungen von Kreatin ist stets diese Monohydratform enthalten. So beschreibt die WO 99/00122 die Herstellung eines Kreatin-Granulats durch Mischen von Kreatin-Monohydrat mit einer wässrigen Polyvinylpyrrolidon-Lösung und weiteren Hilfsstoffen. Das feuchte Granulat wird bei 45°C getrocknet und zu Tabletten verpresst. Das Verfahren weist den Nachteil auf, dass zur Gewinnung von Tabletten mehrere zeit- und kostenaufwendige Schritte notwendig sind. Die erhaltenen Tabletten weisen bei einem Tablettengewicht von 135 mg einen Gehalt an Kreatin-Monohydrat von 100 mg, entsprechend 5,0 mmol/g Kreatin Tablettengewicht, auf.

Im Handel sind ferner Kreatin-Dosierungsformen in Form von Gelatine-Steckkapseln mit einem Gehalt an Kreatin-Monohydrat von etwa 70%, entsprechend 4,7 mmol Kreatin, bezogen auf die Masse der Kapsel in g, erhältlich. Die Herstellung und das Abfüllen der Gelatine-Steckkapseln sind ebenfalls zeit- und kostenaufwendig.

Der Kreatingehalt dieser bekannten, auf Kreatin-Monohydrat beruhenden Dosierungsformen kann nicht ohne weiteres erhöht werden, da der Anteil des Bindemittels bzw. der Kapselhülle am Gesamtgewicht nicht unter einen kritischen Wert gesenkt werden kann, ohne die mechanischen Eigenschaften der Dosierungsform zu verschlechtern. Kreatin-Monohydrat enthält 12 Gew.-% Kristallwasser. Da das Kristallwasser keine physiologische Wirkung hat, ließen sich feste Dosierungsformen mit einem höheren molaren Kreatingehalt unter Verwendung eines Kreatin-Hypohydrats oder von wasserfreiem Kreatin erhalten. Diese Kreatin-Formen sind jedoch schlecht definiert und außerdem hygroskopisch. Sie würden aus diesem Grund zu Dosierungsformen führen, die an der Umgebungsluft instabil sind.

Seit einiger Zeit ist zur Herstellung fester pharmazeutischer Formen das so genannte Verfahren der Schmelzkalandrierung bekannt, bei dem man eine wirkstoffhaltige, im Wesentlichen lösungsmittelfreie Schmelze aus einem polymeren, wirkstoffhaltigen Bindemittel extrudiert und den extrudierten Strang zu der gewünschten Arzneiform formt, beispielsweise in einem Kalander mit Formwalzen, siehe EP-A-240 904, EP-A-240 906, EP-A-337 256 und EP-A-358 105. Als polymeres Bindemittel werden insbesondere Polymere des N-Vinylpyrrolidons oder Copolymerisate davon z.B. mit Vinylacetat, eingesetzt. Die Ausbildung der wirkstoffhaltigen Schmelze wird hier im Allgemeinen bei einer Temperatur von etwa 150°C erreicht.

Die Anwendung der Schmelzkalandrierung zur Herstellung fester Kreatin-Dosierungformen wurde bisher nicht beschrieben. Bei der Schmelzkalandrierung ist es unvermeidbar, dass der pharmazeutische Wirkstoff der Einwirkung höherer Temperaturen ausgesetzt ist. Da Kreatin bereits bei Temperaturen ab etwa 80°C der thermischen Zersetzung insbesondere zu Kreatinin unterliegt, war die Anwendung der Schmelzkalandrierung zur Herstellung Kreatin enthaltender Dosierungsformen nicht naheliegend.

Überraschenderweise wurde ein Verfahren zur Herstellung fester Kreatin-Dosierungsformen gefunden, das großtechnisch wirtschaftlich anwendbar ist, wenige Verfahrensschritte aufweist und die Herstellung von stabilen Kreatin-Dosierungsformen mit einem hohen molaren Anteil an Kreatin ermöglicht.

Die Erfindung betrifft ein Verfahren zur Herstellung fester Kreatin enthaltender Dosierungsformen, wobei man
a) ein Gemisch herstellt, das wenigstens ein thermoplastisches, physiologisch verträgliches, wasserlösliches oder wasserquellbares polymeres Bindemittel und Kreatin umfasst und 1 bis 20 mol Wasser pro mol Kreatin enthält,
b) das Gemisch bei oder oberhalb des Erweichungspunktes des polymeren Bindemittels vorzugsweise unter zumindest partieller Verdampfung des Wassers plastifiziert,
c) das plastifizierte Gemisch zu Dosierungsformen formt und abkühlt.

Die Erfindung betrifft außerdem die nach dem Verfahren erhältlichen Dosierungsformen. Sie betrifft ferner eine Kreatin enthaltende feste Dosierungsform, umfassend wenigstens 3,3 mmol Kreatin, bezogen auf die Masse der Dosierungsform in g, in feindisperser oder molekulardisperser Verteilung in einer Matrix aus einem thermoplastischen, physiologisch verträglichen, wasserlöslichen oder wasserquellbaren polymeren Bindemittel

Der Begriff "feste Dosierungsform" soll eine insbesondere zur oralen oder rektalen Verabreichung geeignete Darreichungsform beliebiger Gestalt bezeichnen, wie z.B. Tabletten, Dragees, Pastillen, Pellets, Granulat und dergleichen.

Das in Schritt a) hergestellte Gemisch enthält 1 bis 20 mol, vorzugsweise 1 bis 15 mol, insbesondere 3 bis 10 mol, Wasser pro mol Kreatin. Der Wassergehalt setzt sich aus dem als Kristallwasser an Kreatin gebundenen Anteil und dem "freien" Wasser im Gemisch zusammen. Dieses Wasser wirkt vermutlich bei erhöhter Temperatur einerseits als temporäres Plastifizierungsmittel für das wasserlösliche oder wasserquellbare polymere Bindemittel und begrenzt andererseits durch seine Verdampfungsenthalpie die thermische Belastung des Kreatins.

Zur Herstellung des Gemisches kann man von den trockenen, wasserfreien Komponenten ausgehen und diese mit der erforderlichen Menge Wasser versetzen. Zweckmäßiger ist jedoch die Verwendung von Kreatin-Monohydrat, d.h. eines Kreatinhydrats mit 1 mol Kristallwasser pro mol Kreatin. Geeignet sind auch Kreatin-Monohydrat-Formen, die über ihren Gehalt an Kristallwasser hinaus ungebundenes Wasser enthalten, das z.B. als Feuchtigkeit an der Oberfläche der Kristallite anhaftet oder zwischen den Kristallen eingeschlossen ist. Solche Formen werden erhalten, wenn eine wäßrige Suspension von Kreatin-Monohydrat, die in der Regel bei der chemischen Synthese von Kreatin primär erhalten wird, filtriert oder zentrifugiert wird. Der Filtrationsrückstand beziehungsweise Zentrifugationsrückstand enthält z.B. 5 bis 50 Gew.-%, meist 15 bis 30 Gew.-% anhaftendes Wasser, das nicht als Kristallwasser gebunden ist. Der Einsatz des restfeuchten Filtrationsrückstandes beziehungsweise Zentrifugationsrückstandes weist zudem den Vorteil auf, dass eine Trocknung zum Kreatin-Monohydrat nicht erforderlich ist.

Kreatin wird technisch im Allgemeinen durch Guanylierung von Sarkosin; d.h. Übertragung des Guanylrestes (Carbamimidoylrestes) auf Sarkosin oder dessen Salze hergestellt. Als Guanylierungsmittel kommen O-Alkylisoharnstoffsalze, insbesondere O-Methylisoharnstoffmethylsulfat, (vgl. JP 59000, DE 197 48 696 oder DE-A 198 60 048.8) oder Cyanamid (vgl. EP 0754 679) in Betracht.

Das polymere Bindemittel wird in der Regel in einer im Wesentlichen wasserfreien Form, d.h. vorzugsweise nicht als Lösung oder Dispersion, eingesetzt. Viele wasserlösliche oder wasserquellbare polymere Bindemittel nehmen bei Lagerung unter Umgebungsbedingungen Feuchtigkeit auf. Es stellt sich dabei ein Gleichgewichtsfeuchtigkeitsgehalt von z.B. 1 bis 5 Gew.-% ein. Diese Formen werden für die vorliegenden Zwecke als "im Wesentlichen wasserfrei" angesehen.

Beim erfindungsgemäßen Verfahren wird Kreatin feindispers oder molekulardispers in eine Matrix eines wasserlöslichen oder wasserquellbaren polymeren Bindemittels eingebettet, vorzugsweise unter Bildung einer festen Lösung. Auf diese Weise werden Kreatin-Hypohydrate (d.h. Hydrate mit weniger als 1 mol Kristallwasser pro mol Kreatin) beziehungsweise wasserfreies Kreatin stabilisiert, so dass erfindungsgemäß hergestellte Dosierungsformen auch dann unter Umgebungsbedingungen stabil sind, wenn sie pro mol enthaltenem Kreatin weniger als 1 mol Wasser enthalten.

Üblicherweise werden pro mol Kreatin 15 bis 70 g, vorzugsweise 40 bis 70 g, thermoplastisches, physiologisch verträgliches, wasserlösliches oder wasserquellbares polymeres Bindemittel eingesetzt. Vorzugsweise wird die Zusammensetzung des Gemisches so gewählt und/oder beim Plastifizieren eine solche Menge Wasser verdampft, dass das plastifizierte Gemisch wenigstens 3,3 mmol, vorzugsweise wenigstens 4,2 mmol und insbesondere wenigstens 5,1 mmol, z.B. 5,1 bis 5,4 mmol, Kreatin, bezogen auf die Masse des plastifizierten Gemisches in g enthält. Vorzugsweise enthält das plastifizierte Gemisch weniger als 1 mol Wasser pro mol Kreatin. Die genannten Werte gelten entsprechend für die erhaltenen Dosierungsformen.

Wasserlösliche oder wasserquellbare polymere Bindemittel enthalten Einheiten hydrophiler Monomere, gegebenenfalls in Verbindung mit Einheiten hydrophober Monomere. Sie lassen sich unter anderem den natürlichen oder modifizierten Polysacchariden; bei Raumtemperatur festen Polyalkylenoxiden; Homo- und Copolymeren hydrophiler, ethylenisch ungesättigter Monomere, wie N-Vinylamiden, ethylenisch ungesättigten Mono- und Dicarbonsäuren, (Meth)acrylamid, Hydroxyalkyl(meth)acrylaten und dergleichen, zuordnen.

Geeignete Bindemittel sind beispielsweise:

Polyvinylpyrrolidon (PVP), Copolymerisate von N-Vinylpyrrolidon (NVP) und Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkylmethacrylate, Copolymerisate von Methylmethacrylat und Acrylsäure, Polyacrylamide, Polyethylenglykole, Polyvinylformamid (gegebenenfalls partiell oder vollständig hydrolysiert), Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane. Davon sind Polyvinylpyrrolidon, Polyethylenglycol, Copolymere von N-Vinylpyrrolidon und Vinylestern, Polyhydroxyalkylacrylate, Polyhydroxyalkylmethacrylate, Alkylcellulosen und Hydroxyalkylcellulosen besonders bevorzugt, insbesondere die unter der Handelsbezeichnung Kollidon® geführten Polyvinylpyrrolidone und Vinylpyrrolidon-Vinylacetat-Copolymere.

Vorteilhaft für die Verwendung als polymeres Bindemittel sind solche Bindemittel, die einen K-Wert (nach Fikentscher, Cellulose-Chemie 13 (1932), S. 58-64, 71-74) im Bereich zwischen 10 und 100, vorzugsweise zwischen 15 und 80, insbesondere von etwa 30 aufweisen.

Am meisten bevorzugt sind Polyvinylpyrrolidone mit einem K-Wert im Bereich zwischen 20 und 60.

Erfindungsgemäße Dosierungsformen enthalten vorzugsweise wenigstens eines der zuvor beschriebenen polymeren Bindemittel. Sie können darüber hinaus andere Bindemittel enthalten. Über die Art des gewählten Bindemittels oder der Abmischung unterschiedlicher Bindemittel können die Eigenschaften der erfindungsgemäßen festen Kreatin enthaltenden Dosierungsform variiert werden. Insbesondere kann auf diese Weise die Kreatin-Freisetzung gesteuert werden.

Das polymere Bindemittel sollte sich in der Gesamtmischung aller Komponenten im Bereich von 50 bis 150°C, vorzugsweise 60 bis 130°C in einen plastischen Zustand überführen lassen. Die Erweichungstemperatur wird erforderlichenfalls durch übliche pharmakologisch akzeptable weichmachende Hilfsstoffe herabgesetzt. Vorzugsweise enthält das Gemisch aber keinen Weichmacher. Beispiele für derartige Weichmacher sind:
langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylenpropylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat, wovon Polyethylenglycole und Polyethylenpropylenglycole bevorzugt sind.

Die Verwendung eines Weichmachers führt dazu, dass die Erweichungstemperatur des polymeren Bindemittels sinkt. Die Bildung des plastischen Gemisches und die Formgebung können somit bei niedrigeren Temperaturen erfolgen, wodurch der Molekulargewichtsabbau des Polymers und die thermische Zersetzung des Kreatins eingeschränkt wird. Falls verwendet, werden die Weichmacher in einer Menge von weniger als 30 Gew.-%, in der Regel 1 bis 15 Gew.-%, bezogen auf die Polymerkomponente eingesetzt.

In bevorzugten Ausführungsformen enthalten die erfindungsgemäß hergestellten Dosierungsformen wenigstens einen Zuckeralkohol, wie z.B. Mannit, Sorbit, Xylit und insbesondere Isomalt. Der Zukkeralkohol wird vorzugsweise in einer Menge von 10 bis 60 g, insbesondere von 15 bis 45 g pro mol Kreatin eingesetzt. Die Mitverwendung eines Zuckeralkohols erlaubt eine definierte Einstellung der Schmelzviskosität des plastifizierten Gemisches im angegebenen Temperaturbereich. Der Zuckeralkohol wirkt ferner als Solubilisierungsmittel und führt zu einer rascheren Kreatin-Freisetzung.

Erfindungsgemäß erhaltene Dosierungsformen können pharmazeutisch akzeptable Hilfsstoffe enthalten. Solche Hilfsstoffe können die Herstellung der Dosierungsform erleichtern und/oder deren Eigenschaften modulieren. Übliche galenische Hilfsstoffe, deren Gesamtmenge bis zu 100 Gew.-%, bezogen auf das polymere Bindemittel, betragen kann, sind z.B.
die oben erwähnten Weichmacher;
Streckmittel bzw. Füllstoffe, wie Silikate oder Kieselerde, Magnesiumoxid, Aluminiumoxid, Titanoxid, Methylcellulose, Natrium-Carboxymethylcellulose, Talkum, Saccharose, Lactose, Getreide- oder Maisstärke, Kartoffelmehl, insbesondere in einer Konzentration von 0,02 bis 50, vorzugsweise 0,20 bis 20 Gew.-%, bezogen auf das Gesamtgewicht des Gemisches;
Schmiermittel, Gleitmittel und Trennmittel wie Magnesium-, Aluminium- und Calciumstearat, Talkum und Silicone, sowie tierische oder pflanzliche Fette, insbesondere in hydrierter Form und solche, die bei Raumtemperatur fest sind. Diese Fette haben vorzugsweise einen Schmelzpunkt von 30°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Schmier- und Trennmitteln beträgt vorzugsweise 0,1 bis 10 Gew.-%;
Fließmittel, z.B. Kieselerden, insbesondere die unter der Handelsbezeichnung Aerosil® geführten hochreinen Siliziumdioxide, in einer Menge von 0,1 bis 5 Gew.-%, bezogen auf das Gesamtgewicht des Gemisches;
Sprengmittel, z.B. Natriumstärkeglycolat;
Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;
Stabilisatoren, wie Antioxidantien, Lichtstabilisatoren, Hydroperoxid-Vernichter, Radikalfänger, Stabilisatoren gegen mikrobiellen Befall;
Ferner können Netz-, Konservierungs-, Adsorptions- und Formentrennmittel sowie Tenside, vorzugsweise anionische und nicht-ionische, wie z.B. Seifen und seifenähnliche Tenside, Alkylsulfate und -sulfonate, Salze von Gallensäuren, alkoxylierte Fettalkohole, alkoxylierte Alkylphenole, alkoxylierte Fettsäuren und Fettsäureglycerinester, die alkoxyliert sein können, und Solubilisierungsmittel, wie Cremophor (polyethoxyliertes Ricinusöl), Gelucire, Vitamin E-TPGS und Tween (ethoxylierte Sorbitanfettsäureester), zugesetzt werden (vgl. z.B. H. Sucker et al. Pharmazeutische Technologie, Thieme-Verlag, Stuttgart 1978).

Die Auswahl geeigneter Hilfsstoffe beruht auf fachmännischem Wissen, wie es beispielsweise in Fiedler, H.B., Lexikon der Hilfsstoffe für Pharmazie, Kosmetik, und angrenzende Gebiete, 4.Aufl., Aulendorf: ECV-Editio-Cator-Verlag (1996), dargestellt ist.

Beim erfindungsgemäßen Verfahren zur Herstellung fester Kreatin enthaltender Dosierungsformen werden die Komponenten in der Regel zunächst miteinander vermischt. Das Gemisch wird dann vorzugsweise unter zumindest teilweiser Verdampfung des enthaltenen Wassers plastifiziert, d.h. in den plastischen Zustand überführt. Die Reihenfolge der Schritte des Mischens und Plastifizierens ist aber nicht zwingend. Die Herstellung und Plastifizierung des Gemisches können auch fließend ineinander übergehen oder gleichzeitig in hierzu geeigneten Vorrichtungen erfolgen.

Das Plastifizieren des Gemisches erfolgt durch Erwärmen, in der Regel unter zusätzlichem Eintrag mechanischer Energie, z.B. durch Kneten, Vermischen oder Homogenisieren. Vorzugsweise plastifiziert man das Gemisch bei Temperaturen von 50 bis 150°C, besonders bevorzugt von 60 bis 130°C. Das Plastifizieren erfolgt in einer für diesen Zweck üblichen Vorrichtung. Besonders geeignet sind Extruder, die in einem zylindrischen Gehäuse eine oder mehrere mit Knet- und/oder Transportelementen versehene rotierbare Achsen aufweisen. Alternativ kann man beheizbare Behälter mit Rührwerk, z.B. Kneter, einsetzen. Bevorzugt erfolgt die Bildung des plastischen Gemischs jedoch durch Extrusion. Die Verfahrensschritte des Plastifizierens können auf an sich bekannte Art und Weise durchgeführt werden, beispielsweise wie in der EP-A-0 240 904, EP-A-0 337 256, EP-A-0358 108, WO 97/15290 und WO 97/15291 beschrieben. Auf den Inhalt dieser Publikationen und insbesondere die darin enthaltenen Ausführungen zur Schmelzextrusion wird hiermit Bezug genommen.

Das erfindungsgemäße Verfahren erlaubt es, als Extruder Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einzusetzen. Da bei der Extrusion in der Regel zumindest ein Teil des im Gemisch enthaltenen Wassers verdampft wird, sind die Extruder im Allgemeinen mit einem Verdampfungsteil ausgerüstet. Besonders bevorzugt sind Extruder der ZKS-Baureihe von Werner und Pfleiderer.

Die Formung zur Dosierungsform vor dem Erstarren kann in Abhängigkeit von der Viskosität des plastischen Gemischs auf vielfältige Weise erfolgen, z.B. durch Formgießen, Spritzgießen, Pressen, Quetschen oder Kalandrieren. Dazu wird das vorstehend beschriebene plastische Gemisch im erfindungsgemäßen Verfahren einem oder mehreren Formungsschritten zugeführt. Das Zuführen kann durch Pressen, Pumpen, z.B. mit Zahnradpumpen, oder vorzugsweise mit einem Extruder erfolgen.

Der erste Formungsschritt erfolgt vorteilhaft beim Austritt des Extrudats aus dem Extruder durch geeignet geformte Düsen, Blenden oder sonstige Austrittsöffnungen, z.B. durch eine Lochblende, eine Runddüse oder eine Breitschlitzdüse. In der Regel wird so kontinuierlich ein Strangextrudat mit vorzugsweise konstantem Querschnitt, z.B. in Form eines Bandes oder eines Stranges, vorzugsweise mit rundem, ovalem, abgerundetem oder flachem und breitem Querschnitt, erhalten.

Geeignete nachgeschaltete Formungsschritte für Extrudate sind z.B. der Kaltabschlag, d.h. das Schneiden beziehungsweise Zerhakken des Stranges nach zumindest teilweisem Erstarren, der Heißabschlag, d.h. das Zerschneiden beziehungsweise Zerhacken des Stranges in noch plastischer Form oder das Abquetschen des noch plastischen Strangs in einer Quetschvorrichtung. Mit Heiß- oder Kaltabschlag lassen sich z.B. Granulate (Heiß- oder Kaltgranulierung) oder Pellets erhalten. Die Heißgranulierung führt in der Regel zu Dosierungsformen (Tabletten oder Pellets) mit einem Durchmesser von 0,1 bis 10 mm, während die Kaltgranulierung normalerweise zu zylinderförmigen Produkten mit einem Verhältnis von Länge zu Durchmesser von 1 bis 10 und einem Durchmesser von 0,5 bis 10 mm führt.

So können einschichtige, bei Anwendung der Koextrusion aber auch offene oder geschlossene mehrschichtige Arzneiformen hergestellt werden, beispielsweise Oblongtabletten, Dragees, Pastillen und Pellets.

Die Arzneiformen können in einem nachgeschalteten Verfahrensschritt nach üblichen Methoden, falls erwünscht, mit einem Coating versehen werden. Geeignete Materialien für Filmüberzüge sind die als polymere Bindemittel genannten Polymere, insbesondere Polyacrylate, wie die Eudragit®-Typen, Celluloseester, wie die Hydroxypropylcellulosephthalate, sowie Celluloseether, wie Ethylcellulose, Hydroxypropylmethylcellulose oder Hydroxypropylcellulose und Gelatine. Auch weitere Formungsschritte können sich anschließen, wie z.B. die Arrondierung oder Verrundung der durch den Heiß- oder Kaltabschlag erhaltenen Pellets mittels Arrondiervorrichtungen, wie in der DE-A-196 29 753 beschrieben.

In einer geeigneten Ausführungsform wird das plastische Gemisch zur Formung einem Formkalander zugeführt. Geeignete Formkalander weisen in der Regel wenigstens zwei Formwalzen und/oder Bänder auf, wobei mindestens eine der Formwalzen und/oder mindestens eines der Bänder Vertiefungen zur Aufnahme und Formung des plastischen Gemischs aufweist. Vorzugsweise verwendet man einen Formkalander mit paarweise gegenläufig rotierenden Formwalzen, wovon wenigstens eine auf ihrer Mantelfläche Vertiefungen zur Aufnahme und Formung des plastifizierten Gemischs aufweist. Mittels dieser Formkalander lassen sich Granulate und Tabletten beliebiger Größe und Gestalt herstellen. Geeignete Formkalander sind beispielsweise in der EP-A-0 240 904, EP-A-0 240 906 und WO 96/19962, sowie in der EP-A-0 358 105 offenbart, auf die diesbezüglich Bezug genommen wird.

Die erfindungsgemäßen Dosierungsformen finden z.B. Anwendung als Arzneimittel zur Therapie von Krankheiten im Zusammenhang mit mangelhafter Kreatinspeicherung bzw. mit vermehrter -Ausscheidung. Darüberhinaus eignen sich die erfindungsgemäßen Kreatinenthaltenden Dosierungsformen als Nahrungsergänzungsmittel zur Leistungssteigerung im Sport, insbesondere im Kraftsport.

Die vorliegende Erfindung soll anhand der folgenden Beispiele näher veranschaulicht, aber nicht beschränkt werden.

Die in den Beispielen verwendeten Materialien Kreatin-Monohydrat (BASF), Isomalt F (Palatinit), Kollidon®K30 (Polyvinylpyrrolidon oder Polyvidon oder PVP; BASF), Explotab (Natriumstärkeglykolat; Mendell, Patterson, New York) sind Handelsprodukte.

### Beispiel 1:

500 mg-Oblong-Tabletten, die 5,1 mmol Kreatin pro 1,0 g Tablettengewicht enthalten, wurden wie folgt hergestellt:

784 g Kreatin-Monohydrat, 200 g Isomalt F und 100 g Kollidon®K30 (Polyvinylpyrrolidon) wurden 1 Minute lang homogen vermischt, danach extrudiert und zu 500 mg-Oblong-Tabletten kalandriert. Die Kalandrierung der extrudierten Schmelze erfolgte wie in der EP-A 240 904 beschrieben.

Die Extrusion erfolgte unter folgenden Bedingungen:

| | |
|---|---|
| Schuss 1 | 44°C |
| Schuss 2 | 69°C |
| Schuss 3 | 120°C |
| Schuss 4 | 115°C |
| Schuss 5 | 110°C |
| Düse | 114°C |

Die Freisetzung des Wirkstoffs aus den Tabletten wurde nach dem Paddle-Modell (USA, pH change) untersucht. Sie betrug nach 1 h 60%, nach 2 h 80% und nach 6 h 98%.

### Beispiel 2:

784 g Kreatin-Monohydrat, 200 g Isomalt F und 80 g Kollidon®K30 (Polyvinylpyrrolidon) und 20 g Explotab wurden 1 Minute lang homogen vermischt, danach extrudiert und zu 500 mg-Oblong-Tabletten kalandriert.

Die Extrusion erfolgte unter folgenden Bedingungen:

| | |
|---|---|
| Schuss 1 | 37°C |
| Schuss 2 | 55°C |
| Schuss 3 | 130°C |
| Schuss 4 | 119°C |
| Schuss 5 | 120°C |
| Düse | 115°C |

Die Freisetzung des Wirkstoffs aus den Tabletten wurde nach dem Paddle-Modell (USA, pH change) untersucht. Sie betrug nach 1 h 75%, nach 2 h 90% und nach 6 h 99%.

Bei allen Beispielen lagen Nebenprodukte aus Umlagerungs- bzw. Zersetzungsreaktionen (z.B. Kreatinin) unter der Nachweisgrenze von 0,5%, bezogen auf den Gehalt an Kreatin.

## Patentansprüche

1. Verfahren zur Herstellung fester Kreatin enthaltender Dosierungsformen, wobei man
a) ein Gemisch herstellt, das wenigstens ein thermoplastisches, physiologisch verträgliches, wasserlösliches oder wasserquellbares polymeres Bindemittel und Kreatin umfasst und 1 bis 20 mol Wasser pro mol Kreatin enthält,
b) das Gemisch im Bereich von 50 bis 150°C unter zumindest partieller Verdampfung des Wassers mittels eines Extruders bei oder oberhalb des Erweichungspunktes des polymeren Bindemittels plastifiziert,
c) das plastifizierte Gemisch zu Dosierungsformen formt und abkühlt.

2. Verfahren nach Anspruch 1, **dadurch gekennzeichnet, dass** das plastifizierte Gemisch wenigstens 3,3 mmol Kreatin, bezogen auf die Masse des plastifizierten Gemisches in g, enthält.

3. Verfahren nach einem der vorhergehenden Ansprüche, **dadurch gekennzeichnet, dass** das plastifizierte Gemisch mittels eines Formkalanders mit paarweise gegenläufig rotierenden Formwalzen, wovon wenigstens eine auf ihrer Mantelfläche Vertiefungen zur Aufnahme und Formung des plastifizierten Gemisches aufweist, zu Dosierungsformen geformt wird.

4. Verfahren nach einem der vorhergehenden Ansprüche, **dadurch gekennzeichnet, dass** das Bindemittel unter Polyvinylpyrrolidon, Polyethylenglycol, Copolymeren von N-Vinylpyrrolidon und Vinylestern, Polyhydroxyalkylacrylaten, Polyhydroxyalkylmethacrylaten, Alkylcellulosen, Hydroxyalkylcellulosen und Gemischen davon ausgewählt ist.

5. Verfahren nach einem der vorhergehenden Ansprüche, **dadurch gekennzeichnet, dass** das Gemisch wenigstens einen Zuckeralkohol enthält.

6. Kreatin enthaltende feste Dosierungsform, erhältlich nach dem Verfahren nach einem der Ansprüche 1 bis 5.

7. Kreatin enthaltende feste Dosierungsform, umfassend wenigstens 3,3 mmol Kreatin, bezogen auf die Masse der Dosierungsform in g, in feindisperser oder molekulardisperser Verteilung in einer Matrix aus einem thermoplastischen, physiologisch verträglichen, wasserlöslichen oder wasserquellbaren polymeren Bindemittel.

## Claims

1. A process for producing solid creatine-comprising dosage forms, wherein
a) a mixture which comprises at least one thermoplastic, physiologically tolerated, water-soluble or water-swellable polymeric binder and creatine, and comprises 1 to 20 mol of water per mol of creatine is prepared,
b) the mixture is plasticated in the range from 50 to 150°C with at least partial evaporation of the water using an extruder at or above the softening point of the polymeric binder,
c) the plasticated mixture is shaped to dosage forms and cooled.

2. The process according to claim 1, wherein the plasticated mixture comprises at least 3.3 mmol of creatine based on the mass of the plasticated mixture in g.

3. The process according to any of the preceding claims, wherein the plasticated mixture is shaped to dosage forms by means of a molding calender with pairs of counter-rotating molding rolls, at least one of which has on its outer surface depressions for receiving and shaping the plasticated mixture.

4. The process according to any of the preceding claims, wherein the binder is selected from polyvinylpyrrolidone, polyethylene glycol, copolymers of N-vinylpyrrolidone and vinyl esters, poly(hydroxyalkyl acrylates), poly(hydroxyalkyl methacrylates), alkylcelluloses, hydroxyalkylcelluloses and mixtures thereof.

5. The process according to any of the preceding claims, wherein the mixture comprises at least one sugar alcohol.

6. A creatine-comprising solid dosage form obtainable by the process according to any of claims 1 to 5.

7. A creatine-comprising solid dosage form comprising at least 3.3 mmol of creatine, based on the weight of the dosage form in g, in fine dispersion or molecular dispersion in a matrix composed of a thermoplastic, physiologically tolerated, water-soluble or water-swellable polymeric binder.

## Revendications

1. Procédé de production de formes galéniques solides contenant de la créatine, où
a) on produit un mélange qui comprend au moins un liant polymère thermoplastique, physiologiquement acceptable, soluble dans l'eau ou gonflable dans l'eau et de la créatine et qui contient 1 à 20 mol d'eau par mole de créatine,
b) on plastifie le mélange dans le domaine de 50 à 150°C avec évaporation au moins partielle de l'eau au moyen d'une extrudeuse au point de ramollissement du liant polymère ou au-dessus du point de ramollissement du liant polymère,
c) on met en forme le mélange plastifié en formes galéniques et on le refroidit.

2. Procédé selon la revendication 1, **caractérisé en ce que** le mélange plastifié contient au moins 3,3 mmol de créatine, par rapport à la masse du mélange plastifié en g.

3. Procédé selon l'une des revendications précédentes, **caractérisé en ce que** le mélange plastifié est mis en forme en formes galéniques au moyen d'une calandre de mise en forme avec des rouleaux de mise en forme tournant en sens inverse par paires, dont l'un au moins comporte sur sa surface d'enveloppe des évidements pour recevoir et mettre en forme le mélange plastifié.

4. Procédé selon l'une des revendications précédentes, **caractérisé en ce que** le liant est choisi parmi la polyvinylpyrrolidone, le polyéthylèneglycol, les copolymères de N-vinylpyrrolidone et d'esters de vinyle, les poly(acrylates d'hydroxyalkyle), les poly(méthacrylates d'hydroxyalkyle), les alkylcelluloses, les hydroxyalkylcelluloses et leurs mélanges.

5. Procédé selon l'une des revendications précédentes, **caractérisé en ce que** le mélange contient au moins un alcool de sucre.

6. Forme galénique solide contenant de la créatine pouvant être obtenue selon le procédé selon l'une des revendications 1 à 5.

7. Forme galénique solide contenant de la créatine comprenant au moins 3,3 mmol de créatine, par rapport à la masse de la forme galénique en g, en répartition finement dispersée ou dispersée à l'échelle moléculaire dans une matrice constituée par un liant polymère thermoplastique, physiologiquement acceptable, soluble dans l'eau ou gonflable dans l'eau.
